# EUROPEAN PATENT SPECIFICATION

(11) **EP 0 499 193 B1**
(45) Date of publication and mention of the grant of the patent: **24.01.1996**
(21) Application number: 92102227.3
(22) Date of filing: 11.02.1992
(51) Int. Cl.: A61B 3/13, A61B 3/14

(54) **Eye observation apparatus**
Augenbeobachtungsgerät
Appareil d'observation d'oeil

(30) Priority: 15.02.1991 JP 6232/91 U; 16.01.1992 JP 5509/92
(43) Date of publication of application: 19.08.1992
(73) Proprietor: TOMEY CORPORATION, Nagoya-shi, Aichi-ken (JP)
(72) Inventor: Mizuno, Seinosuke, c/o Tomey Corporation, Nagoya-shi, Aichi-ken (JP); Yamada, Yoshihiko, c/o Tomey Corporation, Nagoya-shi, Aichi-ken (JP)
(74) Representative: Türk, Gille, Hrabal, Leifert

(56) References cited:
- DE-A- 2 916 590
- DE-A- 3 031 822
- GB-A- 2 049 216
- US-A- 3 614 214

## Description

The invention relates to an eye observation apparatus, and more particularly to an eye observation apparatus wherein a tip portion can come into contact with an eye to observe and photograph each portions of the eye such a vitreous body, retinal vessel corneal and corneal epithelium.

A known conventional eye observation apparatus of this kind is disclosed in JP-B-36 969/1983 (a patent family member of which is US-A-4 209 225).

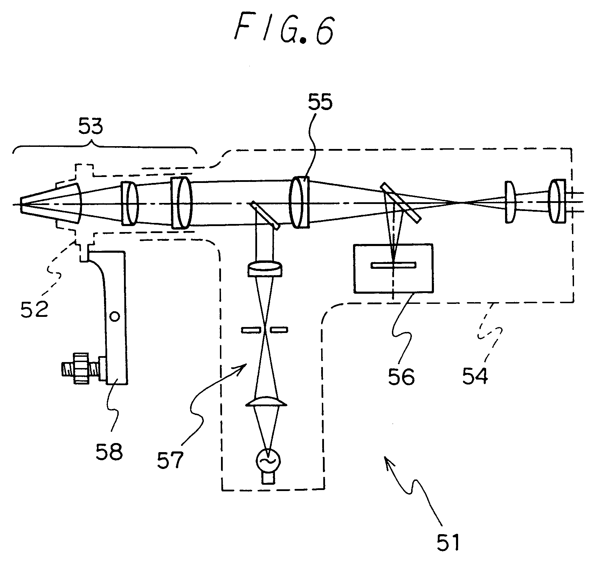

This known observation apparatus 51-as shown in Fig. 6 hereof- comprises a lens barrel 52, a main body 54, an illumination optics 57 and a biasing means 58. An object lens unit 53 stored in the lens barrel 52 is movable relative to the main body 54 along its optical axis. The biasing means 58 biases the lens barrel 52 in direction of an eye so that a tip portion of the object lens unit can constantly contact the eye to be observed. Even if the eye, which is object of inspection, moves, follow-up movement of the object lens unit following the movement of the eye prevents out of focus.

Accordingly, a focusing system is equipped in the object lens unit since it is necessary that a collimated image ray which is emitted from the object lens unit reaches an image lens group unit 55.

Further, in the conventional observation apparatus, a plurality of covered condutors is connected between the object lens unit and the main body as electrical cable for supplying electric power with the focusing system and as electrical cable for electric signales.

However, when covered conductors are used, it is difficult that the eye is coming into contact with the tip portion of the object lens unit due to a reaction force which is generated by bending the covered conductors when the movable portion is moved.

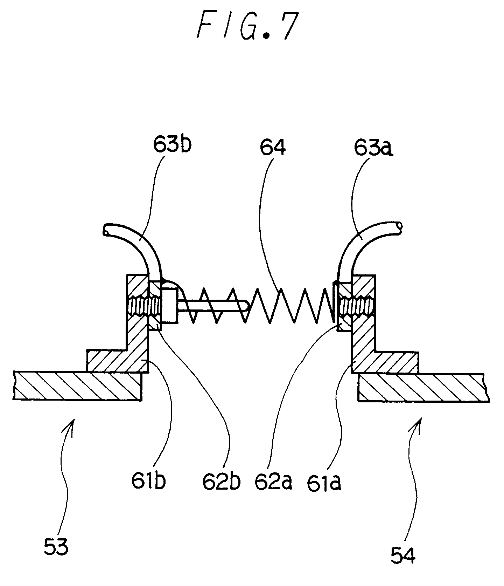

Therefore, a coil 64 of bare conductor which has a good flexibility and small diameter has recently been used instead of covered conductors. That is to say, a covered conductor 63a is connected to a fixed portion of the main body 54 via an insulating plate 61a and an insulating substrate 62a as shown in Fig. 7 hereof. A further covered conductor 63b is connected to the movable portion, i.e. the object lens unit 53 via an insulating plate 61b and an insulating substrate 62b.

The covered conductor 63a is soldered to an end portion of the coil 64 and the covered conductor 63b is soldered to the other end portion of the coil 64 so as to be conducted. One covered conductor 63a is connected to a power source (not shown) and the other covered conductor 63b is connected to a driving portion (not shown) of the object lens unit 53. A plurality of bare wire coil units are used.

The conventional observation apparatus 51 mentioned hereinbefore requires troublesome work as to position the optical axis of the movable object lens unit 53 and the optical axis of the fixed main body 54. The conventional observation apparatus further requires to collimate the image ray and to provide the collimated image ray with the image lens unit 55. Therefore, a focusing system is required to be equipped in the object lens unit 53. However, the location of equipping such focusing system is limited.

The object of the present invention is to avoid the above-mentioned problems and to provide an eye observation apparatus of which is very easy to assemble and in which the focusing system can be arranged at will in the object lens unit, the image lens unit or the image plane portion.

This object is solved by an eye observation apparatus having the features of claim 1. Improvements are subject of the dependent claims.

In this eye observation apparatus the assembly is very easy since there is no need of positioning the optical axis of the movable portion of the apparatus and the fixed portion thereof.

The observation apparatus of the present invention has such a construction that the whole photographing means including a photographic optics, an eyepiece and a camera can be moved on a frame of a main body. Therefore, out of focus (displacement of focus) does not happen due to the follow-up movement of the whole photographing means even if the eye which is object of inspection moves slightly.

Hereinafter, the eye observation apparatus of the present invention is explained with referring to the attached drawings, wherein
- Fig. 1: is an optical-path diagram showing one embodiment of the eye observation apparatus of the present invention,
- Fig. 2: is an optical-path diagram illustrating the focal distance from an imaging lens group in the eye observation apparatus of Fig. 1,
- Fig. 3.: is an optical path diagram illustrating the focal distance from an imaging lens unit in an example of a conventional eye observation apparatus,
- Fig. 4: is an illustrating view showing an embodiment of a biasing device used in the eye observation apparatus of the present invention,
- Fig. 5: is a perspective view showing a flexible electrical cable of Fig. 1 more in detail,
- Fig. 6: is an optical-path diagram showing an embodiment of a conventional eye observation apparatus, and
- Fig. 7: is a front view showing an example of conventional wiring for electric connection in a known eye observation apparatus.

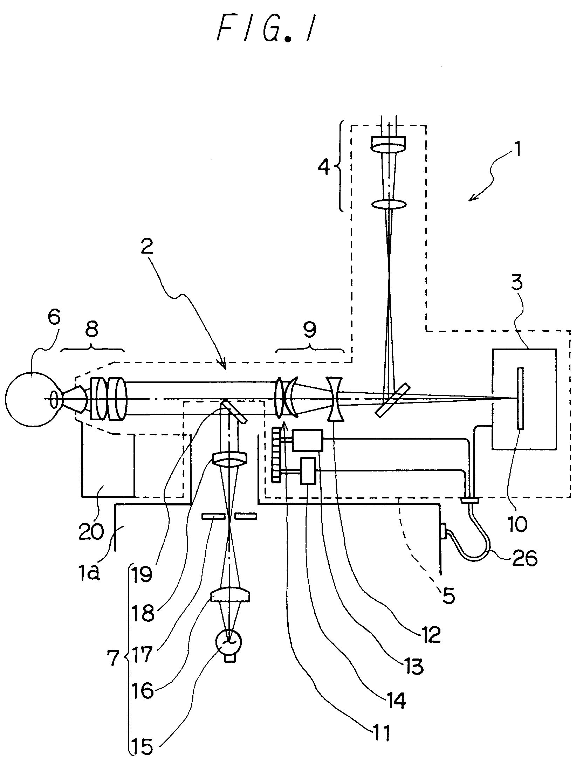

The eye observation apparatus 1 of Fig. 1 comprises a photographing means 5 including a photographic optics 2, a camera 3 and an eyepiece 4, which are movably supported on a frame 1a of a main body. An illumination optics 7 for illuminating the eye 6 which is object of inspection is fixed to the frame 1a. Since the whole photographing means 5 can be moved, positioning the optical axis of a movable portion of an object lens group 8 and the optical axis of a fixed portion of the main body is not required. Therefore, assembling the apparatus is easy.

The moving mechanism of the photographing means 5 has such a construction as normally used so that friction due to movement of the photographing means 5 is reduced as much as possible. For example, said mechanism is sometimes composed of a guiding barrel fixed to the frame 1a, into which the photographing means 5 is inserted, and plural steel balls enclosed between the guiding barrel and the photographing means 5.

The photographic optics 2 comprises the object lens group 8 and an imaging lens group 9 spaced apart from one another. The imaging lens group 9 has the function that image rays transmitted through the object lens group 8 is imaged on an image plane 10 in the cameras 3.

The imaging lens group 9 is composed of a convex lens sub-group 11, the object lens group 8, and a concave lens 12, located on the side of the image plane 10, the convex lens sub-group 11 being spaced apart fom the concave lens 12. The convex lens sub-group 11 has the function of focusing by movement thereof along the optical axis.

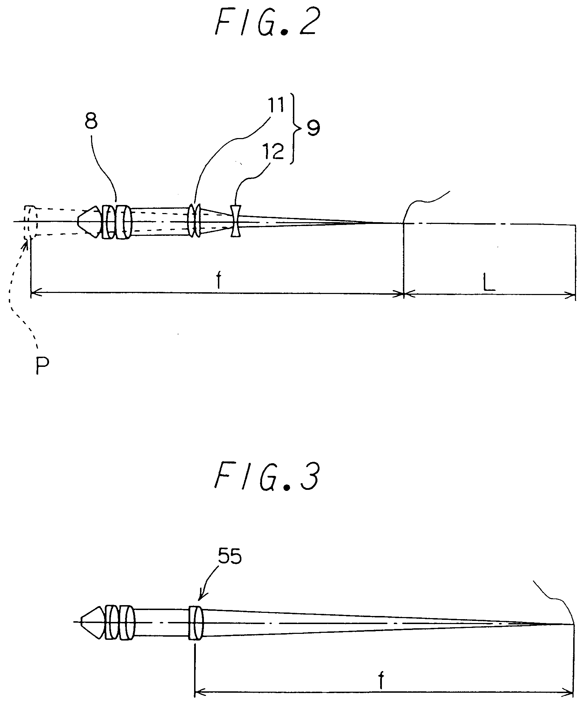

As in the imaging lens group 9 the convex lens sub-group 11 is spaced apart from the concave lens 12, the total length of the apparatus 1 can be short. That is to say, an image ray is converged by the convex lens sub-group 11 of the imaging lens group 9 and then diverged (i.e. the convergent angle is caused to be small) by the concave lens 12 so that the image ray is imaged on th image plane 10 as shown in Fig. 2. In this case, if the focal length of the imaging lens group 9 is set to the same focal length "f" as that of the conventional observation apparatus 51 shown in Fig. 3 (the imaging lens unit 55 is a convex lens unit), the situation of the observation apparatus 1 is the same as if the imaging lens unit 55 is located in the position "p". Then, the distance between the imaging lens group 9 and the image plane 10 of the observation apparatus of the present invention is shorter than that of a conventional observation apparatus by "L". Thereby, the length along the optical axis of the photographing means 5 becomes shorter than that of the conventional observation apparatus. As result, the eye observation apparatus 1 is compact. Accordingly, it becomes easier to move the whole photographing means 5 (hereinafter referred to as a "full-floating").

Furthermore, since the total length of the photographing means can be short without shortening the focal length of the object lens group 8 and the focal length of the imaging lens group 9 (without making lens power higher), fading of an image due to out of focus is reduced.

According to the present invention, full-floating can be perfomred as mentioned herewinbefor. The image ray transmitted from the object lens group 8 to the imaging lens group 9 is not required to be collimated exactly. Therefore, the photographing means 5 can be provided with the focusing system even in the imaging lens group 9.

For focusing it may also be employed a system in which the convex lens sub-group 11 is moved by a miniature motor 13 with a reducer. At the same time, the rotating angle of the motor 13 is detected by an encoder 14 connected with the miniature motor 13.

The illumination optics 7 is fixed to the frame 1a of the main body and has such a construction that a light source for observation 15 is adjacent to a condensing lens 16, the condensing lens 16 is adjacent to a lens stop 17. The lens stop 17 is adjacent to a collimating lens 18, and the collimating lens 18 is adjacent to a releting mirror 19. The refleting mirror 19 is arranged between the object lens group 8 and the imaging lens group 9 in the photographic optics 2. As a result, the photographic optics 2 and the illumination optics 7 share the object lens group 8.

Furthermore, the frame 1a of the main body is provided with a biasing means 20 for constantly biasing the movable photographing means 5 along the optical axis thereof in such a manner that a tip portion of the object lens group 8 is coming into contact with an eye 6, which is object of inspection. By virtue of the biasing means 20, out of focus does not happen since the tip portion of the object lens group 8 can move constantly and follow even if the face of a patient moves or location of the cornea moves due to a change of intraocular pressure or pulse pressure.

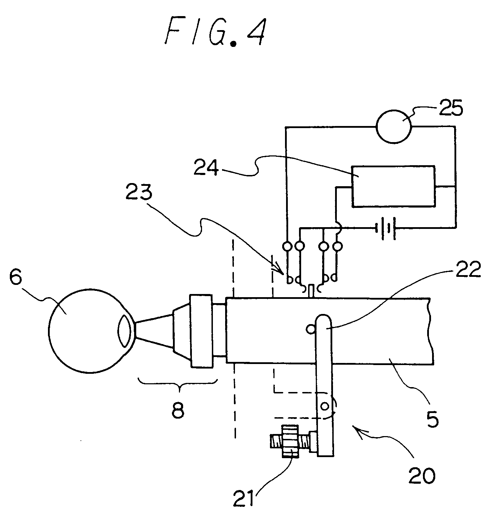

As biasing means 20, a means such that the eye 6 is constantly contacted with the tip portion of the object lens group 8 by weak and constant force can be used. For instance, in such a biasing means with reference to Fig. 4 and disclosed in US-A-4 209 225, a lever 22 exerts moments imposed by an adjustable weight 21, thereby the tip portion of the object lens group 8, is coming into contact with the eye 6. When the eye 6 is pushed excessively by the tip portion of the object lens group 8 a switch 23 operates and a buzzer 24 whistles, and when the eye 6 is parted from the object lens group 8 excessively, a lamp 25 lights.

As biasing means, there is a biasing means (not shown) disclosed in JP-A-142007/1983 or JP-A-39703/1989 wherein the eye 6 is contacted with the tip portion of the object lens group 8 by means of an extension spring.

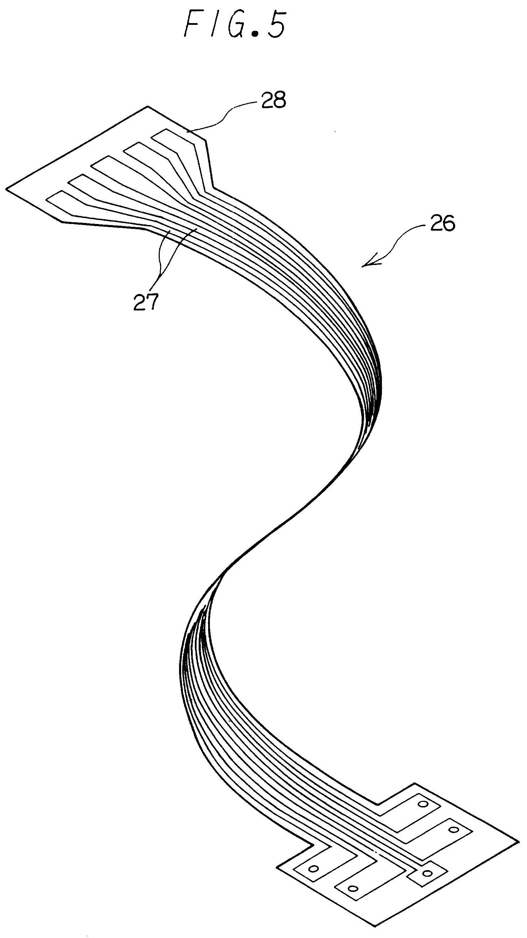

In the eye observation apparatus 1, a flexible electrical cable 26 (flexible flat cable "FFC" or flexible print circuit "FPC") which connects the frame 1a with the photographing means 5 is employed as cable for supplying electric power to the focusing system, the biasing means 20 or a CCD image sensor in the camera 3. The flexible electrical cable 26 is well-known. For example, as shown in Fig. 5, the flexible elctrical cable 27 comprises a plurality of tinned phosphor bronze foil wires coated in such a manner as to be sandwitched by polyvinyl cloride films 28.

For the cable 26, a film 28 such as of polyvinyl chloride which on one side adheres to the tinned phosphor bronze foil wires 27, can be used. Furthermore, a cable, wherein the wire portion is formed by deposition or printing, can also be used.

Flexible electrical cables have the advantage that wiring plural systems collectively can be performed, even in narrow space, and connection with machinery is easy. Flexible electrical cables can be used also in case that the required number of conductors is low.

Moreover, in the present embodiment, the flexible electrical cable is - in consideration of good flexibility of the cable - employed in order to reduce reaction forces generated by bending the cable when the tip portion of the object lens group 8 is coming into contact with an eye 6. That is to say, the reaction force generated by bending the flexible electrical cable 26 hardly comes out when the tip portion of the object lens group 8 is forced to contact with a proper force by moving the object lens group 8. Therefore, the eye is not badly affected by the object lens group 8.

Next, the operation and use of the eye observation apparatus 1 of the embodiment as mentioned hereinbefore is explained.

First, an illuminating ray emitted from a light source for observation 15 of the illumination optics 7 is transmitted through the condensing lens 16, the lens stop 17 and the collimating lens 18 so that the illuminating ray becomes collimated. Then the collimated ray is transmitted through the object lens group 8 by reflection by the reflecting mirror 19, and illuminates the inspection portion of the eye 6.

Next, the image ray emitted from the inspection portion of the eye 6 is collimated to some extent by the object lens group 8 and imaged on the image plane 10 by the imaging lens group 9. In this embodiment the convex lens sub-group 11 has the focusing function and the object lens group 8 does not have a focusing function. Therefore, the image ray emitted from any part of the eye is not a perfectly collimated ray. However, there is no problem since the focus can be checked by moving the convex lens sub-group 11 along the optical axis. Further, if the focus is once checked, out of focus does not happen since the whole photographing means 5 including the image plane 10 is movable.

In the embodiment, the eye observation apparatus 1 has such a focusing system that the convex lens sub-group 11 of the image lens group 9 can be moved.

Accoding to the present invention, the focusing system is not limited to the above embodiment. There can be such a focusing system that the concave lens 12 can be moved or a part of the object lens group 8 can be moved. Also, the image plane 10 can be provided with a focusing system.

When the image of the inspection portion of the eye is enlarged with high magnification in order to observe endothelium cornea or corneal epithelium, the length of a conventional observation apparatus in the direction of its optical axis must be large. On the contrary, in the here described embodiment the imaging lens group 9, which has such a construction that the convex lens sub-group 11 is apart from the concave lens 12, can considerable shorten the distance between the image lens group 9 and the image plane 10 compared with an imaging lens which is composed of only the convex lens sub-group 11. As result, the photographing means 5 can be easily moved along the optical axis thereof, i.e. full-floating can be easily performed as mentioned hereinbefore.

Moreover, the eye observation apparatus can be conveniently handled since the whole eye observation apparatus is compact.

With the eye observation apparatus of the present invention it is not necessary to position the optical axis, so that the apparatus is easy to assemble, and the location of setting the focusing system can be designed more freely than in a conventional eye observation apparatus.

## Claims

1. Eye observation apparatus, comprising
* a main body;
* an illumination optics (7) which is fixed on a frame (1a) of the main body, said illumination optics illuminating a portion under inspection of an eye (6) to be examined;
* a photographing means (5), including
** an imaging optics (2) for causing an image ray which is emitted from said portion under inspection to reach an image plane (10); and
* a biasing means (20) for biasing the photographing means (5) so as to establish constant contact between the imaging optics (2) and the eye (6);
**characterized** in that
* the imaging optics (2) comprises an object lens group (8) and an imaging lens group (9), which latter consists of a convex lens sub-group (11) and a concave lens (12), wherein said convex lens sub-group (11) is positioned spaced apart from the concave lens (12) towards the object lens group (8); and
* the photographing means (5) is arranged movably within the frame (1a) for axial displacement along the optical axis of the imaging optics (2).

2. Apparatus of claim 1, characterized in that either the convex lens sub-group (11) or the concave lens (12) is movable along the optical axis of the imaging optics (2).

3. Apparatus of claim 1, characterized in that the photographing means (5) has an electrical equipment, and a flexible electrical cable (26) for electrical connection is equipped between the photographing means (5) and the frame (1a) of the main body.

4. Apparatus of claim 3, characterized in that the flexible electrical cable (26) comprises a plurality of foil wires (27) having electric conductivity and a sythetic resin film (28), the synthetic resin film being attached to one side of the plurality of foil wires.

## Patentansprüche

1. Augenbeobachtungsgerät, mit
- einem Hauptkörper;
- einer Ausleuchteoptik (7), die auf einem Rahmen (1a) des Hauptkörpers befestigt ist, wobei die Ausleuchteoptik einen zu überprüfenden Teil eines Auges (6), das untersucht werden soll, ausleuchtet;
- einer Photographiereinrichtung (5), welche
-- eine Abbildeoptik (2) zum Bewirken, daß ein Abbildungsstrahl, der von dem zu überprüfenden Teil ausgesendet wird, eine Bildebene (10) erreicht, umfaßt; und
- einer Vorbelastungseinrichtung (20) zum Vorbelasten der Photographiereinrichtung (5), um so einen konstanten Kontakt zwischen der Abbildeoptik (2) und dem Auge (6) einzurichten;
dadurch gekennzeichnet, daß
- die Abbildeoptik (2) eine Objekt-Linsengruppe (8) und eine abbildende Linsengruppe (9) aufweist, wobei die letztere aus einer Konvexlinsen-Untergruppe (11) und einer Konkavlinse (12) besteht, wobei die Konvexlinsen-Untergruppe (11) von der Konkavlinse (12) in Richtung auf die Objekt-Linsengruppe (8) beabstandet angeordnet ist; und
- die Photographiereinrichtung (5) bewegbar innerhalb des Rahmens (1a) für die axiale Verlagerung entlang der optischen Achse der Abbildeoptik (2) angeordnet ist.

2. Gerät nach Anspruch 1, dadurch gekennzeichnet, daß die Konvexlinsen-Untergruppe (11) oder die Konkavlinse (12) entlang der optischen Achse der Abbildeoptik (2) bewegbar ist.

3. Gerät nach Anspruch 1, dadurch gekennzeichnet, daß die Photographiereinrichtung (5) eine elektrische Ausstattung hat und ein flexibles elektrisches Kabel (26) für die elektrische Verbindung zwischen der Photographiereinrichtung (5) und dem Rahmen (1a) des Hauptkörpers angeordnet ist.

4. Gerät nach Anspruch 3, dadurch gekennzeichnet, daß das flexible elektrische Kabel (26) eine Vielzahl von Folienleitungen (27) mit elektrischer Leitfähigkeit und einen Kunstharzfilm (28) aufweist, wobei der Kunstharzfilm an einer Seite der Vielzahl der Folienleitungen befestigt ist.

## Revendications

1. Appareil d'observation d'oeuil, comprenant
- un corps principal;
- un moyen optique d'éclairage (7), fixé sur un cadre (1a) du corps principal, ledit moyen d'éclairage éclairant une partie sous inspection d'un oeuil (6) à examiner;
- un moyen de photographie (5), y compris
-- un moyen optique d'image (2) pour causer un rayon d'image émis de ladite partie sous inspection à arriver à un plan focal (10); et
- un moyen de charge (20) pour charger ledit moyen de photographie (5) à but d'établir un contact constant entre ledit moyen optique d'image (2) et l'oeuil (6);
caractérisé en ce que
- ledit moyen optique d'image (2) comprends un groupe de lentille d'objet (8) et un groupe de lentille d'image (9), ce dernier étant constitué par un sous-groupe de lentille convexe (11) et une lentille concave (12), ledit sous-groupe de lentille convexe (11) étant positionné séparé de ladite lentille concave (12) vers ledit groupe de lentille d'objet (8); et
- ledit moyen de photographie (5) est disposé à manière mobile dans le cadre (1a) pour le déplacement axial le long de l'axe optique du moyen optique d'image (2).

2. Appareil selon la revendication 1, caractérisé en ce que le sous-groupe de lentille convexe (11) ou la lentille concave (12) peut être mis en mouvement le long de l'axe optique du moyen optique d'image (2).

3. Appareil selon la revendication 1, caractérisé en ce que le moyen de photographie (5) comprends un équipement électrique, et un flexible câble électrique (26) pour la connexion électrique est prévu entre le moyen de photographie (5) et le cadre (1a) du corps principal.

4. Appareil selon la revendication 3, caractérisé en ce que le flexible câble électrique (26) comprends une multiplicité des fils de ruban (27) avec conductibilité électrique et un film de résine synthétique (28), le film de résine synthétique étant fixé à un côté de la multiplicité des fils de ruban.
